# EUROPÄISCHE PATENTSCHRIFT

(11) **EP 4 225 113 B1**
(45) Veröffentlichungstag und Bekanntmachung des Hinweises auf die Patenterteilung: **28.10.2026**
(21) Anmeldenummer: 21787321.5
(22) Anmeldetag: 29.09.2021
(51) Int. Cl.: A47K 5/12, A47K 10/32, B05B 11/00

(54) **MULTIFUNKTIONSSPENDER ZUM AUSGEBEN VON REINIGUNGSAKTIVEN FLÜSSIGKEITEN**
MULTIFUNCTION DISPENSER FOR DISPENSING CLEANING-ACTIVE LIQUIDS
DISTRIBUTEUR MULTIFONCTION POUR DISTRIBUER DES LIQUIDES À ACTION NETTOYANTE

(30) Priorität: 05.10.2020 DE 102020006088
(43) Veröffentlichungstag der Anmeldung: 16.08.2023
(73) Patentinhaber: Mercedes-Benz Group AG, 70372 Stuttgart (DE)
(72) Erfinder: FISCHER, Jan, 72072 Tübingen (DE)
(74) Vertreter: Novagraaf Group
(86) Internationale Anmeldenummer: PCT/EP2021/076798
(87) Internationale Veröffentlichungsnummer: WO 2022/073822

(56) Entgegenhaltungen:
- GB-A- 2 525 457
- JP-A- 2014 234 217
- US-B1- 8 517 171

## Beschreibung

Flüssigkeiten nach dem Anspruch 1.

Ein Multifunktionsspender der eingangs genannten Art ist in der Druckschrift GB 2 525 457 A und in der Druckschrift US 8 517 171 B1 beschrieben.

Solche Multifunktionsspender sind bspw. aus der Druckschrift DE 37 44 438 A1 bekannt, wonach ein entsprechender Multifunktionsspender aus einem verschlossenen ersten Gehäuseteil für Tücher, Vliese und Papiere und einem daran längsseits einstückig angeformten, separat verschließbaren, zweiten Gehäuseteil für Flüssigkeiten, Pulver oder Pasten und einem Flüssigkeitsspender für diese Mittel besteht. Nachteilig an diesem Multifunktionsspender ist, dass das erste und zweite Gehäuseteil relativ großbauend sind, wodurch der bekannte Multifunktionsspender relativ sperrig ist.

In der Druckschrift JP 2014 234217 A ist ein weiterer Multifunktionsspender offenbart.

Die Aufgabe der Erfindung liegt daher darin, eine verbesserte oder zumindest eine andere Ausführungsform eines Multifunktionsspenders anzugeben. Insb. soll versucht werden, einen vergleichsweise kompakten Multifunktionsspender bereitzustellen.

Bei der vorliegenden Erfindung wird diese Aufgabe insb. durch den Gegenstand des unabhängigen Anspruchs gelöst. Vorteilhafte Ausführungsformen sind Gegenstand der abhängigen Ansprüche und der Beschreibung.

Der Grundgedanke der Erfindung liegt darin, das erste und zweite Gehäuseteil sowie den Multifunktionsspender aufeinanderzustapeln, wodurch der vorgeschlagene Multifunktionsspender relativ kompakt und platzsparend ist.

Hierzu ist ein Multifunktionsspender zum Ausgeben von reinigungsaktiven Flüssigkeiten vorgesehen, welcher mit einem sich in einer Hauptausdehnungsrichtung, insb. entlang einer geraden Mittenachse, ersteckenden, mehrteiligen Gehäuse, das ein zum Aufnehmen von Medizinprodukten geeignetes, einstückiges, erstes topfförmiges Gehäuseteil und ein daran, d.h. am ersten Gehäuseteil, berührend angeordnetes, zum Aufnehmen von reinigungsaktiven Flüssigkeiten geeignetes, einstückiges, zweites topfförmiges Gehäuseteil aufweist. Reinigungsaktive Flüssigkeiten können bspw. Desinfektionsmittel oder Seifen oder Duftstoffmittel sein. Reinigungsaktive Flüssigkeiten können nach dem Verständnis der Erfindung sowohl flüssig, pulverförmig als auch pastös vorliegen. Medizinprodukten sind zweckmäßigerweise Hygienemitteln wie Gesichtsmasken, Taschentücher, Reinigungsmittel oder Desinfektionsmittel. Jedenfalls definiert das erste Gehäuseteil in seiner Hauptausdehnungsrichtung eine gerade Erstmittenachse und weist einen Bestückungsdurchgang zum Durchführen von Medizinprodukten auf. Mind. ein Medizinprodukt ist durch den Bestückungsdurchgang hindurch in das erste Gehäuseteil eingelegt. Das zweite Gehäuseteil definiert in seiner Hauptausdehnungsrichtung eine gerade Zweitmittenachse und bevorratet reinigungsaktive Flüssigkeit. Der Multifunktionsspender hat weiterhin einen manuell betätigbaren Flüssigkeitsspender zum Ausgeben von reinigungsaktiven Flüssigkeiten. Dieser definiert wiederum in seiner Hauptausdehnungsrichtung eine gerade Spendermittenachse. Wenn der Flüssigkeitsspender, insb. manuell, betätigt ist, wird ein vorgegebenes Volumen oder eine vorgegebene Masse von reinigungsaktiver Flüssigkeit aus dem zweiten Gehäuseteil gefördert und, insb. nach außerhalb vom Gehäuse hin, ausgegeben. Wesentlich für die Erfindung ist, dass insb. die Mittenachse, die Spendermittenachse, die Erstmittenachse und die Zweitmittenachse zueinander koaxial ausgerichtet sind. Dadurch liegen die Komponenten des Multifunktionsspenders hinsichtlich ihrer jeweiligen Achsen auf einer gemeinsamen Linie, wodurch sich eine kompakte Gestalt des Multifunktionsspenders realisieren lässt. Das ist insb. dann von Vorteil, wenn das erste Gehäuseteil und das zweite Gehäuseteil rotationssymmetrisch bezüglich ihrer jeweiligen Achse sind. Ferner kann der Flüssigkeitsspender entweder am ersten Gehäuseteil und/oder am zweiten Gehäuseteil fixiert sein, so dass ein alle besagten Komponenten des Flüssigkeitsspenders eine Baueinheit bilden. Zweckmäßigerweise können das erste Gehäuseteil und das zweite Gehäuseteil lösbar aneinander angeordnet sein. Bspw. kann dies durch eine Schraubverbindung, eine Schnappverbindung, einen Schnellverschluss oder eine Klemmverbindung zwischen dem ersten Gehäuseteil und dem zweiten Gehäuseteil realisiert sein. Das hat den Effekt, dass die beiden separaten Gehäuseteile zerstörungsfrei voneinander gelöst und ohne weitere Hilfsmittel wieder aneinander festgemacht werden können.

Erfindungsgemäß ist weiterhin, dass der Flüssigkeitsspender, wenn er manuell betätigt ist, ein vorgegebenes Volumen oder eine vorgegebene Masse von reinigungsaktiver Flüssigkeit aus dem zweiten Gehäuseteil fördern und diese nicht nur ausgeben, sondern entweder in das erste Gehäuseteil zum Benetzen des darin angeordneten mindestens einen Medizinprodukts einleiten oder, insbesondere nach außerhalb vom Gehäuse hin, ausgeben kann. Der Flüssigkeitsspender hat dabei einen sich entlang der Spendermittenachse erstreckenden rundzylindrischen Spenderschaft zum Durchleiten von reinigungsaktiven Flüssigkeiten, wobei einenends am Spenderschaft, insb. am freien Ende des Spenderschafts, ein, insb. von außen, weiter insb. von außerhalb des Gehäuses her, zugänglicher gehäuseaußenliegender, knaufförmiger Spenderkopf des Flüssigkeitsspenders angeordnet ist, von dem ein bezüglich der Spendermittenachse winkelig, insb. rechtwinkelig, wegragender innenhohler, mit dem Spenderschaft fluidisch kommunizierender Spenderschnabel zum Ausgeben von reinigungsaktiven Flüssigkeiten angeordnet ist. Der Spenderschaft durchsetzt dabei das erste Gehäuseteil und das zweite Gehäuseteil jeweils zumindest abschnittsweise. Der Begriff "knaufförmig" beschreibt zweckmäßigerweise einen kugel-, ovoid- oder knopfförmigen Körper. Am Spenderkopf kann ferner eine, insbesondere parallel bezüglich der Spendermittenachse ausgerichtete, Betätigungskraft zum Betätigen des Flüssigkeitsspenders eingeleitet werden, bspw. manuell von Hand, so dass reinigungsaktive Flüssigkeit aus dem zweiten Gehäuseteil durch den Spenderschaft hindurch zum Spenderschnabel gefördert bzw. gepumpt werden kann. Das hat zunächst den Vorteil, dass sich der Flüssigkeitsspender relativ bequem von einem Nutzer benutzen lässt. Ferner ist dadurch insgesamt ein Multifunktionsspender bereitgestellt, welcher einerseits eine reinigungsaktive Flüssigkeit, insb. Desinfektionsmittel oder Seifen, bspw. an einen Nutzer ausgeben kann, sowie welcher andererseits mit derselben Flüssigkeit ein in den Multifunktionsspender innen eingelegtes Medizinprodukt, insb. Hygienemitteln wie Gesichtsmasken, Taschentüchern, Reinigungsmitteln oder Desinfektionsmitteln, benetzen und/oder beduften kann. Das hat den Vorteil, dass eine Doppelfunktion bereitgestellt ist, wodurch auf zwei separate, jeweils eine Funktion realisierende Vorrichtungen verzichtet werden kann.

Zweckmäßigerweise kann zwischen dem Spenderkopf des Flüssigkeitsspenders und dem Gehäuse ein in Richtung der Spendermittenachse mehrere Faltenberge werfender Faltenbalg angeordnet sein. Der Faltenbalg kann einenends am ersten oder zweiten Gehäuseteil und anderenends am Spenderkopf berührend lose angelegt oder berührend fixiert angelegt sein.

Weiter zweckmäßigerweise kann der Flüssigkeitsspender berührend am ersten Gehäuseteil fixiert sein, wobei der Spenderschaft das erste Gehäuseteil vollständig und das zweite Gehäuseteil abschnittsweise durchsetzt. Alternativ kann der Flüssigkeitsspender berührend am zweiten Gehäuseteil fixiert sein, wobei der Spenderschaft dann das erste Gehäuseteil abschnittsweise und das zweite Gehäuseteil vollständig durchsetzt. Insb. kann dabei vorgesehen sein, dass der Spenderkopf des Flüssigkeitsspenders an einer exponierten, für einen Nutzer einfach zugänglichen Stelle steht, wobei in Richtung der Spendermittenachse an den Spenderkopf das erste Gehäuseteil und dann das zweite Gehäuseteil folgt. Alternativ kann auch vorgesehen sein, dass der Spenderkopf des Flüssigkeitsspenders an einer exponierten, für einen Nutzer einfach zugänglichen Stelle steht, wobei in Richtung der Spendermittenachse an den Spenderkopf zunächst das zweite Gehäuseteil und dann das erste Gehäuseteil angeschlossen ist. Mit anderen Worten können das erste und zweite Gehäuseteil in Richtung der Spendermittenachse auch in umgekehrter Reihenfolge angeordnet sein. Das hat den Vorteil, dass der vorgeschlagene Multifunktionsspender relativ flexibel an unterschiedlichste Einbausituationen, bspw. in der Mittelkonsole eines Kraftfahrzeuges, anpassen lässt.

Zweckmäßigerweise kann der Flüssigkeitsspender einen innerhalb des ersten Gehäuseteils angeordneten gehäuseinnenliegenden Desinfektionsmittelausgabeabschnitt zum Einleiten von reinigungsaktiven Flüssigkeiten in das erste Gehäuseteil zum Benetzen des im ersten Gehäuseteil angeordneten mindestens einen Medizinprodukts aufweisen. Der Desinfektionsmittelausgabeabschnitt kann dabei am Spenderschaft angeordnet sein. Insb. kann einenends am freien Ende des Spenderschafts ein von außen, insb. von außerhalb des Gehäuses, her zugänglicher gehäuseaußenliegender knaufförmiger Spenderkopf des Flüssigkeitsspenders und mitteninne, bspw. auf halber Spenderschaftlänge, am Spenderschaft der Desinfektionsmittelausgabeabschnitt angeordnet sein. Das hat den vorteilhaften Effekt, dass, wenn am Spenderkopf eine Betätigungskraft zum Betätigen des Flüssigkeitsspenders eingeleitet wird, bspw. manuell von Hand, reinigungsaktive Flüssigkeit aus dem zweiten Gehäuseteil durch den Spenderschaft hindurch zum Desinfektionsmittelausgabeabschnitt oder zum Spenderschnabel gefördert bzw. gepumpt werden kann. Dadurch können im ersten Gehäuseteil angeordnete Medizinprodukte mit reinigungsaktiver Flüssigkeit benetzt und dadurch bspw. desinfiziert werden. Das hat den Vorteil, dass sich entsprechende Medizinprodukte mittels des Flüssigkeitsspenders relativ bequem von einem Nutzer benetzen bzw. desinfizieren lassen.

Weiterhin kann der Desinfektionsmittelausgabeabschnitt aus einer mehrere Sprühöffnungen aufweisenden Öffnungsgruppe gebildet sein. Zweckmäßigerweise kann der Spenderschaft des Flüssigkeitsspenders einen hohlzylindrischen Kreisrohrkörper haben, an dessen nicht mit seiner Mantelfläche gleichzusetzenden stofflichen Rohrmantel die Sprühöffnungen der Öffnungsgruppe angeordnet oder ausgebildet sind. Zweckmäßig kann sein, wenn man die Sprühöffnungen dabei jeweils in Richtung der Spendermittenachse mit identischen Längsabstand zueinander und in einer um die Spendermittenachse rotierenden Umfangsrichtung mit identischen Umfangsabstand zueinander anordnet. Dadurch wird gewährleistet, dass die in den Multifunktionsspender eingelegten Medizinprodukte gleichmäßig und vollständig, insb. vollflächig, mit reinigungsaktiven Flüssigkeiten benetzen lassen.

Zweckmäßigerweise kann der Bestückungsdurchgang, insb. in dessen Zusammenbauzustand, in Richtung der Erstmittenachse und der Zweitmittenachse axial zwischen dem ersten Gehäuseteil und dem zweiten Gehäuseteil angeordnet und vom zweiten Gehäuseteil, insb. fluiddicht und/oder vollständig, verschließbar oder verschlossen sein. Dadurch ist das die Medizinprodukte aufnehmende erste Gehäuseteil durch das zweite Gehäuseteil so verschlossen oder verschließbar, dass die besagten Medizinprodukte unverlierbar im ersten Gehäuseteil gelagert werden. Dadurch ergibt sich der Vorteil, dass die Medizinprodukte nicht verloren gehen und dass auf einen separaten Deckel zum Abdecken des ersten Gehäuseteils verzichtet werden kann. Dadurch ist der Multifunktionsspender leichtbauend und aufgrund der damit einhergehenden relativ geringen Gesamtbauteilanzahl kostengünstig.

Weiter zweckmäßigerweise ist der Spenderkopf des Flüssigkeitsspenders, insb. manuell, drehbeweglich am Spenderschaft angeordnet und zwischen exakt zwei oder exakt drei oder mind. drei voneinander abweichenden Drehstellungen um die Spendermittenachse herum hin und her rotierbar. Der Flüssigkeitsspender ist, wenn der Spenderkopf in eine als Sperrstellung bezeichnete erste Drehstellung dieser Drehstellungen rotiert ist, in welcher der Spenderkopf eine Bezugswinkelposition, insb. von exemplarisch 0°, definiert, in Richtung der Spendermittenachse nicht betätigbar, sondern gesperrt, so dass mittels des Flüssigkeitsspenders reinigungsaktive Flüssigkeit weder über den Desinfektionsmittelausgabeabschnitt in das erste Gehäuseteil eingeleitet noch über den Spenderschnabel ausgegeben werden kann. Ferner ist der Flüssigkeitsspender, wenn der Spenderkopf in eine als Spendestellung bezeichnete zweite Drehstellung dieser Drehstellungen rotiert ist, in welcher der Spenderkopf eine im Winkelbereich von +10° bis +90° gegenüber der Bezugswinkelposition abweichende Spendewinkelposition definiert, in Richtung der Spendermittenachse durch Beaufschlagung mit Betätigungskraft, bspw. manuell von Hand, des Spenderkopfs betätigbar, so dass mittels des Flüssigkeitsspenders reinigungsaktive Flüssigkeit durch den Spenderschaft gefördert, insb. gepumpt, und über den Spenderschnabel ausgegeben ist, bspw. auf eine Hand des Nutzers. Sofern drei oder mind. drei Drehstellungen vorgesehen sind, ist der Flüssigkeitsspender, wenn der Spenderkopf in eine als Benetzungsstellung bezeichnete dritte Drehstellung dieser Drehstellungen rotiert ist, in welcher der Spenderkopf eine im Winkelbereich von -10° bis -90° gegenüber der Bezugswinkelposition abweichende Benetzungswinkelposition definiert, in Richtung der Spendermittenachse durch Beaufschlagung mit Betätigungskraft, bspw. manuell von Hand, des Spenderkopfs betätigbar, so dass mittels des Flüssigkeitsspenders reinigungsaktive Flüssigkeit aus dem zweiten Gehäuseteil durch den Spenderschaft des Flüssigkeitsspenders hin zum Desinfektionsmittelausgabeabschnitt durchgeleitet und das im ersten Gehäuseteil angeordnete mind. eine Medizinprodukte benetzt ist. Auch hierdurch ist ein Multifunktionsspender bereitgestellt, welcher einerseits eine reinigungsaktive Flüssigkeit, insb. Desinfektionsmittel oder Seifen, bspw. an einen Nutzer ausgeben kann sowie welcher andererseits mit derselben Flüssigkeit ein in den Multifunktionsspender innen eingelegtes Medizinprodukt, insb. Hygienemitteln wie Gesichtsmasken, Taschentüchern, Reinigungsmitteln oder Desinfektionsmitteln, benetzen und/oder beduften kann. Der Spenderkopf lässt sich dabei bspw. manuell von Hand zwischen den besagten Drehstellungen hin und her schalten. Das hat den Vorteil, dass eine bequem zu kontrollierende Doppelfunktion bereitgestellt ist.

Weiterhin kann das erste Gehäuseteil und/oder das zweite Gehäuseteil in eine Mittelkonsole eines Kraftfahrzeuges integriert sind. Dadurch kann der Multifunktionsspender einfach in ein Kraftfahrzeug integriert und für den Kraftfahrzeugnutzer bequem zugänglich in selbigem positioniert werden.

Zweckmäßigerweise kann im Inneren des ersten Gehäuseteils ein Korb oder ein Gitter zum Aufnehmen und Spannen des mindestens einen Medizinprodukts angeordnet sein. Weiterhin können die besagten Sprühöffnungen der Öffnungsgruppe jeweils durch einen Zerstäuber gebildet sein. Ferner kann der besagte Bestückungsdurchgang einen zum manuellen Einlegen und manuellen Entnehmen mind. eines Medizinprodukts in das erste Gehäuseteil geeigneten Runddurchgang aufweisen oder diesen bilden. Es kann auch vorgesehen sein, dass der besagte Bestückungsdurchgang eine geschlitzte, insb. kreuz- oder sternförmig geschlitzte, Ausgabeeinrichtung oder einen einschlitzförmigen Durchgang aufweist oder bildet, die jeweils zum Durchführen und Ausgeben mind. eines im ersten Gehäuseteil bevorrateten Medizinprodukts geeigneten sind.

Zusammenfassend bleibt festzuhalten: Die vorliegende Erfindung betrifft einen Multifunktionsspender zum Ausgeben von reinigungsaktiven Flüssigkeiten mit einem Gehäuse, das ein zum Aufnehmen von Medizinprodukten geeignetes, erstes topfförmiges Gehäuseteil und ein daran angeordnetes, zum Aufnehmen von reinigungsaktiven Flüssigkeiten geeignetes, zweites topfförmiges Gehäuseteil aufweist. Das erste Gehäuseteil definiert eine Erstmittenachse, weist einen Bestückungsdurchgang zum Durchführen von Medizinprodukten auf und bevorratet mind. ein Medizinprodukt, welches durch den Bestückungsdurchgang in das erste Gehäuseteil eingelegt ist. Das zweite Gehäuseteil definiert eine Zweitmittenachse und bevorratet reinigungsaktive Flüssigkeit. Der Multifunktionsspender ist weiterhin mit einem eine Spendermittenachse definierenden betätigbaren Flüssigkeitsspender zum Ausgeben von reinigungsaktiven Flüssigkeiten ausgerüstet. Wesentlich für die Erfindung ist, dass der Flüssigkeitsspender, wenn er betätigt ist, ein vorgegebenes Volumen oder eine vorgegebene Masse von reinigungsaktiver Flüssigkeit aus dem zweiten Gehäuseteil fördert und entweder in das erste Gehäuseteil zum Benetzen des darin angeordneten mindestens einen Medizinprodukts einleitet oder an einen Nutzer ausgibt. Die Spendermittenachse, die Erstmittenachse und die Zweitmittenachse sind dabei zueinander koaxial ausgerichtet.

Weitere wichtige Merkmale und Vorteile der Erfindung ergeben sich aus den Unteransprüchen, aus den Zeichnungen und aus der zugehörigen Figurenbeschreibung anhand der Zeichnungen.

Bevorzugte Ausführungsbeispiele der Erfindung sind in den Zeichnungen dargestellt und werden in der nachfolgenden Beschreibung näher erläutert, wobei sich gleiche Bezugszeichen auf gleiche oder ähnliche oder funktional gleiche Bauteile beziehen.

Dabei zeigen, jeweils schematisch:
- Fig. 1: in einer Teilschnittansicht ein bevorzugtes Ausführungsbeispiel eines erfindungsgemäßen Multifunktionsspenders,
- Fig. 2: in einer weiteren Teilschnittansicht den Multifunktionsspender aus Fig. 1, allerdings sind die beiden Gehäuseteile des Multifunktionsspenders nun zueinander beabstandet angeordnet, um in das erste Gehäuseteil ein Medizinprodukt entnehmen oder einlegen zu können,
- Fig. 3: in einer perspektivischen Ansicht den Multifunktionsspender aus Fig. 1, wobei exemplarisch drei Drehstellungen des Spenderkopfs des Flüssigkeitsspenders illustriert sind, ferner
- Fig. 4: in einer perspektivischen Ansicht ein bevorzugtes weiteres Ausführungsbeispiel eines erfindungsgemäßen Multifunktionsspenders, weiterhin
- Fig. 5: in einer perspektivischen Ansicht kein bevorzugtes weiteres Ausführungsbeispiel eines erfindungsgemäßen Multifunktionsspenders und zuletzt
- Fig. 6: in einer perspektivischen Ansicht den Multifunktionsspender aus Fig. 5, allerdings sind die beiden Gehäuseteile des Multifunktionsspenders nun zueinander beabstandet angeordnet, um aus dem ersten Gehäuseteil ein Medizinprodukt entnehmen zu können.

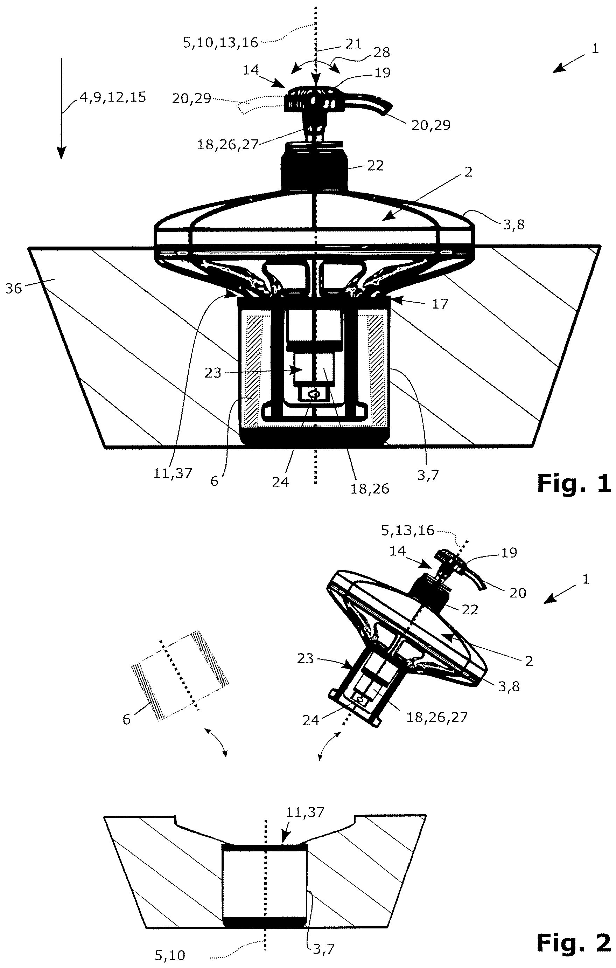

Die Fig. 1 bis 6 zeigen ein im Gesamten mit dem Bezugszeichen 1 bezeichneten Multifunktionsspender, welcher zum Ausgeben von reinigungsaktiven Flüssigkeiten 2 dient. Derartige reinigungsaktive Flüssigkeiten 2 sind bspw. Desinfektionsmittel oder Seifen oder Duftstoffmittel, welche exemplarisch flüssig, pulverförmig als auch pastös vorliegen können und allesamt einen reinigenden und/oder desinfizierenden und/oder beduftenden Effekt bereitstellen.

Die Fig. 1 bis 3 zeigen ein bevorzugtes erstes Ausführungsbeispiel eines erfindungsgemäßen Multifunktionsspenders 1, der exemplarisch in eine Mittelkonsole 36 eines Kraftfahrzeuges integriert ist. In Fig. 1 kann man einerseits eine längs aufgetrennte Mittelkonsole 36 eines nicht illustrierten Kraftfahrzeuges erkennen, deren Schnittflächen sind schraffiert dargestellt. Die Mittelkonsole 36 stellt eine Aufnahmemulde zur Aufnahme des Multifunktionsspenders 1 bereit, in welche ein einziger Multifunktionsspender 1 berührend eingesteckt und bspw. durch Verklemmen, Verschrauben oder Verkleben fixiert ist, um im Betrieb des Kraftfahrzeuges festen Halt und Vibrationsfreiheit sicherzustellen.

Der Multifunktionsspender 1 zum Ausgeben von reinigungsaktiven Flüssigkeiten 2 nach den Fig. 1 bis 3 hat ein mehrteiliges Gehäuse 3, das sich exemplarisch in einer Hauptausdehnungsrichtung 4 entlang einer geraden Mittenachse 5 erstreckt. Das mehrteilige Gehäuse 3 weist ein zum Aufnehmen von Medizinprodukten 6, wie bspw. Gesichtsmasken, Taschentüchern, Reinigungsmitteln oder Desinfektionsmitteln, geeignetes, einstückiges, erstes topfförmiges Gehäuseteil 7 und ein daran berührend angeordnetes, zum Aufnehmen von reinigungsaktiven Flüssigkeiten 2 geeignetes, einstückiges, zweites topfförmiges Gehäuseteil 8 auf. Das insb. aus Kunststoff hergestellte, erste Gehäuseteil 7 definiert in seiner Hauptausdehnungsrichtung 9 eine gerade Erstmittenachse 10 und weist einen Bestückungsdurchgang 11 zum Durchführen von Medizinprodukten 6 auf, exemplarisch handelt es sich um einen Runddurchgang 37. Gemäß Fig. 1 ist im Inneren des ersten Gehäuseteils 7 exemplarisch ein einziges Medizinprodukt 6 eingelegt. In Fig. 1 und 2 kann man ferner erkennen, dass das erste Gehäuseteil 7 integral in die Mittelkonsole 36 eingearbeitet oder daran angeordnet ist, was die Integration des Multifunktionsspenders 1 in ein Kraftfahrzeug begünstigt und es für einen Nutzer bequem zugänglich macht. Das insb. ebenfalls aus Kunststoff hergestellte zweite Gehäuseteil 8 definiert in seiner Hauptausdehnungsrichtung 12 eine gerade Zweitmittenachse 13 und bevorratet seinerseits im Inneren ein vorgegebenes Volumen oder eine vorgegebene Masse von reinigungsaktiver Flüssigkeit 2. Exemplarisch ist der Bestückungsdurchgang 11, insb. in dessen Zusammenbauzustand, in Richtung der Erstmittenachse 10 und der Zweitmittenachse 13 axial zwischen dem ersten Gehäuseteil 7 und dem zweiten Gehäuseteil 8 angeordnet und vom zweiten Gehäuseteil 8 fluiddicht verschließbar gestaltet. Mit anderen Worten, das zweite Gehäuseteil 8 fungiert wie ein Deckel zum Verschließen des Bestückungsdurchgangs 11 des ersten Gehäuseteils 7.

Wie in Fig. 2 angedeutet, lässt sich das Gehäuseteil 8 vom Gehäuseteil 7 abnehmen, um das Medizinprodukt 6 aus den ersten Gehäuseteil 7 herauszunehmen oder dieses einzulegen. In den Fig. 1 bis 3 sind das erste Gehäuseteil 7 und das zweite Gehäuseteil 8 dabei exemplarisch mittels einer Schraubverbindung 17 lösbar berührend aneinander angeordnet, wodurch die vorteilhafte Option gegeben ist, die beiden separaten Gehäuseteile 7, 8 zerstörungsfrei voneinander zu lösen und wieder aneinander festzumachen.

Der Multifunktionsspender 1 nach den Fig. 1 bis 3 hat weiterhin einen am zweiten Gehäuseteil 8 angeordneten, bspw. aufgeschraubten oder integral angeformten, manuell betätigbaren Flüssigkeitsspender 14, der zum Ausgeben von reinigungsaktiven Flüssigkeiten 2 dient. Er definiert in seiner Hauptausdehnungsrichtung 15 eine Spendermittenachse 16. Wenn der Flüssigkeitsspender 14 betätigt ist oder wird, bspw. durch Beaufschlagung des Flüssigkeitsspenders 14 mittels einer in Fig. 1 durch einen Pfeil angedeuteten, insb. manuell von Hand aufgebrachten, Betätigungskraft 21, ist ein vorgegebenes Volumen oder eine vorgegebene Masse von reinigungsaktiver Flüssigkeit 2 aus dem Inneren des zweiten Gehäuseteils 8 gefördert, insb. gepumpt, und entweder in das erste Gehäuseteil 7 zum Benetzen des darin angeordneten Medizinprodukts 6 eingeleitet oder nach außen hin zum Nutzer ausgegeben. Dadurch ist insgesamt ein Multifunktionsspender 1 bereitgestellt, welcher eine reinigungsaktive Flüssigkeit 2 an einen Nutzer ausgeben und mit derselben Flüssigkeit 2 ein in den Multifunktionsspender 1 innen eingelegtes Medizinprodukt 6 benetzen und/oder beduften kann. Um den Multifunktionsspender 1 kompakt zu gestalten sind exemplarisch insb. die Mittenachse 5, die Spendermittenachse 16, die Erstmittenachse 10 und die Zweitmittenachse 13 zueinander parallel und koaxial ausgerichtet. Dadurch liegen die Komponenten des Multifunktionsspenders 1 zueinander hinsichtlich ihrer jeweiligen Achsen auf einer gemeinsamen Linie und sind parallel. Das ist insb. dann von Vorteil, wenn das erste Gehäuseteil 7 und das zweite Gehäuseteil 8 rotationssymmetrisch bezüglich ihrer jeweiligen Achse gestaltet sind.

In Fig. 1 bis 3 kann man ferner erkennen, dass der Flüssigkeitsspender 14 einen sich entlang der Spendermittenachse 16 erstreckenden durchgängig rundzylindrischen Spenderschaft 18 zum Durchleiten von reinigungsaktiven Flüssigkeit 2 hat. Der Spenderschaft 18 hat exemplarisch einen hohlrundzylindrischen Kreisrohrkörper 26, der einen stofflichen Rohrmantel 27 mit rotierender Mantelfläche aufweist. Der Spenderschaft 18 ist durch das erste Gehäuseteil 7 abschnittsweise und durch das zweite Gehäuseteil 8 vollständig hindurchgeführt. Einenends am Spenderschaft 18 ist ein von außen her vom Nutzer zugänglicher gehäuseaußenliegender knaufförmiger Spenderkopf 19 angeordnet, an dem zweckmäßigerweise die besagte Betätigungskraft 21 zum Pumpbetätigen des Flüssigkeitsspenders 14 eingeleitet werden kann. Am Spenderkopf 19 ist ein mit dem Spenderschaft 18 fluidisch kommunizierender Spenderschnabel 20 angeformt, der bezüglich der Spendermittenachse 16 winkelig, insb. rechtwinkelig, wegragt und zum Ausgeben von reinigungsaktiven Flüssigkeiten 2 dient, wenn der Flüssigkeitsspender 14 insb. mit der besagten Betätigungskraft 21 betätigt wird. In Richtung der Spendermittenachse 16 zwischen dem Spenderkopf 19 und dem zweiten Gehäuseteil 8 ist exemplarisch ein in Richtung der Spendermittenachse 16 mehrere Faltenberge werfender Faltenbalg 22 angeordnet. Daneben hat der Flüssigkeitsspender 14 einen innerhalb des ersten Gehäuseteils 7 angeordneten, gehäuseinnenliegenden, Medizinprodukt 6 zugewandten Desinfektionsmittelausgabeabschnitt 23 zum Einleiten von reinigungsaktiven Flüssigkeiten 2 in das erste Gehäuseteil 7 zum Benetzen des im ersten Gehäuseteil 7 angeordneten Medizinprodukts 6. Der Desinfektionsmittelausgabeabschnitt 23 kann dabei am Spenderschaft 18 integral ausgebildet und mitteninne oder anderenends am Spenderschaft 18 angeordnet sein. Dadurch ist es möglich, wenn der Spenderkopf 19 mit besagter Betätigungskraft 21 beaufschlagt wird, den Flüssigkeitsspender 14 zu betätigen, wobei reinigungsaktive Flüssigkeit 2 aus dem Inneren des zweiten Gehäuseteils 7 durch den Spenderschaft 18 hindurch zum Desinfektionsmittelausgabeabschnitt 23 oder zum Spenderschnabel 20 gefördert bzw. gepumpt wird. Dadurch können im ersten Gehäuseteil 7 angeordnete Medizinprodukte 6 mit reinigungsaktiver Flüssigkeit 2 benetzt und bspw. desinfiziert werden. In Fig. 1 und 2 kann man noch erkennen, dass der Desinfektionsmittelausgabeabschnitt 23 aus einer einzigen am Rohrmantel 27 des Spenderschafts 18 angeordneten Sprühöffnung 24 gebildet ist. Mehr als eine Sprühöffnung 24 ist natürlich auch denkbar. Die Sprühöffnung 24 kann ferner durch einen Zerstäuber gebildet sein.

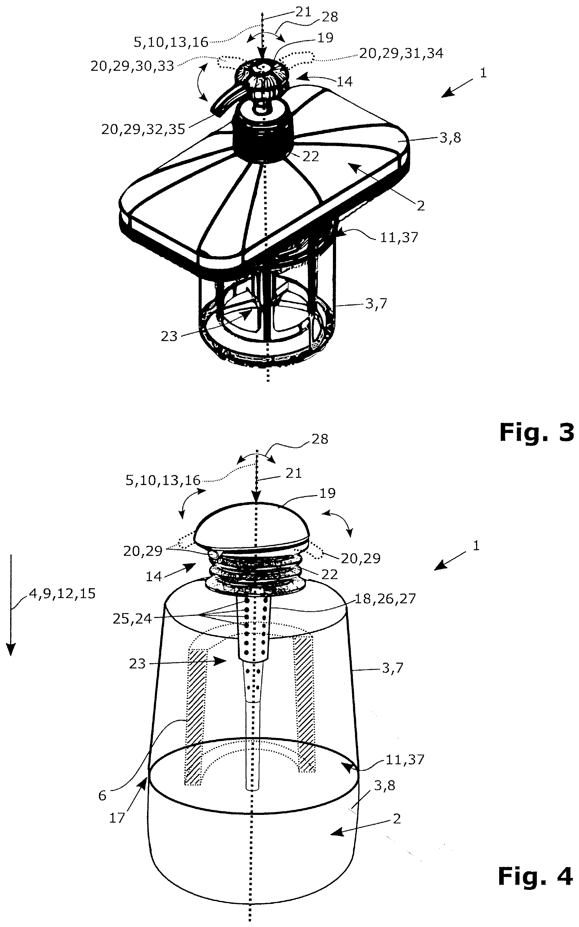

Zuletzt zeigt Fig. 3 den Multifunktionsspender 1 in einer perspektivischen Ansicht, so dass man erkennen kann, dass der besagte Spenderkopf 19 mitsamt des Spenderschnabels 20 zwischen exemplarisch drei, voneinander winkelig abweichenden Drehstellungen 29 in einer um die Spendermittenachse 16 herum rotierenden Umfangsrichtung 28 hin und her rotiert werden kann, bspw. manuell von Hand. Hierzu ist der Spenderkopf 19 drehbeweglich am Spenderschaft 18 oder am ersten oder zweiten Gehäuseteil 7, 8 angeordnet. In Fig. 3 ist angedeutet, dass der Flüssigkeitsspender 14, wenn der Spenderkopf 19 in die illustrierte als Sperrstellung 30 bezeichnete erste Drehstellung dieser Drehstellungen 29 rotiert ist, in Richtung der Spendermittenachse 16 nicht betätigbar sondern bewegungsgesperrt ist. Dadurch kann mittels des Flüssigkeitsspenders 14 reinigungsaktive Flüssigkeit 2 weder über den Desinfektionsmittelausgabeabschnitt 23 in das erste Gehäuseteil 7 eingeleitet noch über den Spenderschnabel 20 zum Nutzer ausgegeben werden. Der Spenderkopf 19 definiert in dieser Drehstellung 29 eine Bezugswinkelposition 33, insb. exemplarisch von 0°.

In Fig. 3 ist mittels punktierten Linien ferner angedeutet, dass der Flüssigkeitsspender 14, wenn der Spenderkopf 19 in eine als Spendestellung 31 bezeichnete zweite Drehstellung dieser Drehstellungen 29 rotiert ist, in Richtung der Spendermittenachse 16 durch Beaufschlagung mit Betätigungskraft 21 des Spenderkopfs 19 in Richtung der Spendermittenachse 16 betätigbar ist. Dadurch kann mittels des Flüssigkeitsspenders 14 eine gewisse Masse bzw. ein gewisses Volumen an reinigungsaktiver Flüssigkeit 2 durch den Spenderschaft 18 gefördert, insb. gepumpt, und über den Spenderschnabel 20 zu einem Nutzer ausgegeben werden. In dieser Drehstellung 29 definiert der Spenderkopf 19 eine sogenannte Spendewinkelposition 34, welche gegenüber der besagten Bezugswinkelposition 33 im Winkelbereich von +10° bis +90° winkelig rotiert ist.

Ferner ist in Fig. 3 ist angedeutet, dass der Flüssigkeitsspender 14, wenn der Spenderkopf 19 in eine als Benetzungsstellung 32 bezeichnete dritte Drehstellung dieser Drehstellungen 29 rotiert ist, in Richtung der Spendermittenachse 16 durch Beaufschlagung mit Betätigungskraft 21 des Spenderkopfs 19 in Richtung der Spendermittenachse 16 betätigbar ist. Dadurch kann mittels des Flüssigkeitsspenders 14 reinigungsaktive Flüssigkeit 2 aus dem zweiten Gehäuseteil 8 durch den Spenderschaft 18 des Flüssigkeitsspenders 14 hindurch zum Desinfektionsmittelausgabeabschnitt 23 durchgeleitet werden, um letztlich das reinigungsaktive Flüssigkeit 2 mithilfe der besagten Sprühöffnung 24 im ersten Gehäuseteil 7 angeordnete eine Medizinprodukte 6 zu benetzen. In dieser Drehstellung 29 definiert der Spenderkopf 19 eine sogenannte Benetzungswinkelposition 35, welche gegenüber der besagten Bezugswinkelposition 33 im Winkelbereich von -10° bis -90° winkelig rotiert ist. Hierdurch kann einerseits eine reinigungsaktive Flüssigkeit 2 an einen Nutzer ausgeben sowie andererseits mit derselben Flüssigkeit 2 ein in den Multifunktionsspender 1 innen eingelegtes Medizinprodukt 6 benetzt und/oder beduftet werden.

Die Fig. 4 zeigt in einer perspektivischen Ansicht ein bevorzugtes weiteres Ausführungsbeispiel eines erfindungsgemäßen Multifunktionsspenders 1. Dieser unterschiedet sich vom Multifunktionsspender 1 gemäß dem vorangehenden Ausführungsbeispiel dadurch, dass der Flüssigkeitsspender 14 nun nicht mehr am zweiten Gehäuseteil 8 sondern am ersten Gehäuseteil 7 angeordnet ist und dass sich dessen Spenderschaft 18 durch das erste Gehäuseteil 7 vollständig und das zweite Gehäuseteil 8 abschnittsweise erstreckt. Jedenfalls ist die vorstehende Funktion des Flüssigkeitsspenders 14 im Wesentlichen unverändert, insb. kann der dortige Spenderkopf 19 ebenfalls in die drei besagten Drehstellung 29 betätigt werden. Am im ersten Gehäuseteil 7 innenliegenden, Medizinprodukt 6 zugewandten, Desinfektionsmittelausgabeabschnitt 23 des Spenderschafts 18 sind nun in Reihen gruppierte Sprühöffnungen 24 vorgesehen, welche zusammen eine Öffnungsgruppe 25 bilden. Es handelt sich bspw. jeweils um Zerstäuber. Exemplarisch sind die Sprühöffnungen 24 jeweils in Richtung der Spendermittenachse 16 mit identischen Längsabstand zueinander und in einer um die Spendermittenachse 16 rotierenden Umfangsrichtung 28 mit identischen Umfangsabstand zueinander anordnet. Dadurch wird gewährleistet, dass das in den Multifunktionsspender 1 eingelegte Medizinprodukt 6 gleichmäßig und vollständig, insb. vollflächig, mit reinigungsaktiven Flüssigkeiten 2 benetzt werden kann. Das das Medizinprodukt 6 aufnehmende erste Gehäuseteil 7 kann zweckmäßigerweise aus einem transparenten Kunststoff gefertigt sein.

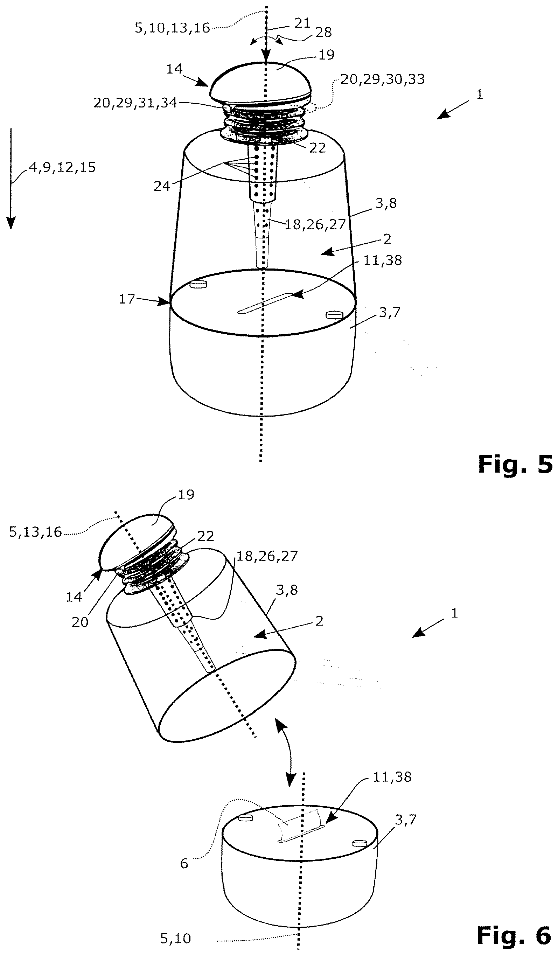

Die Fig. 5 zeigt in einer perspektivischen Ansicht kein bevorzugtes weiteres Ausführungsbeispiel eines erfindungsgemäßen Multifunktionsspenders 1. Es dient wie die vorangehenden Ausführungsbeispiele dazu, reinigungsaktive Flüssigkeiten 2, insb. Desinfektionsmittel oder Seifen, an Nutzer auszugeben. Allerdings können innen eingelegte Medizinprodukte 6 mittels der reinigungsaktiven Flüssigkeiten 2 nicht benetzt werden. Auch dieser Multifunktionsspender 1 kann in eine, in den Fig. 5 und 6 allerdings nicht angedeutete, Mittelkonsole eines Kraftfahrzeuges eingebaut werden. Konkret ist vorgesehen, dass der Multifunktionsspender 1 ein sich in einer Hauptausdehnungsrichtung 4 insb. entlang einer geraden Mittenachse 5 ersteckendes, mehrteiliges aus Kunststoff gefertigtes Gehäuse 3 hat, welches ein zum Aufnehmen von Medizinprodukten 6, insb. Hygienemitteln wie Gesichtsmasken, Taschentüchern, Reinigungsmitteln oder Desinfektionsmitteln, geeignetes, einstückiges, erstes topfförmiges Gehäuseteil 7 und ein daran, d.h. am ersten Gehäuseteil 7, berührend angeordnetes, zum Aufnehmen von reinigungsaktiven Flüssigkeiten 2, insb. auch Pulver oder Pasten, geeignetes, einstückiges, zweites topfförmiges Gehäuseteil 8 aufweist. Das erste Gehäuseteil 7 definiert in seiner Hauptausdehnungsrichtung 9 eine gerade Erstmittenachse 10 und bildet einen Bestückungsdurchgang 11 zum Durchführen von Medizinprodukten 6. Der Bestückungsdurchgang 11 ist vorliegend als einschlitzförmiger Durchgang 38 gestaltet, obwohl auch geschlitzte, insb. kreuz- oder sternförmig geschlitzte, Ausgabeeinrichtungen vorstellbar sind. Der Bestückungsdurchgang 11 kann in dieser Gestalt das Ausgeben von Medizinprodukten 6 kontrollieren, insb. eignet er sich zum Durchführen und Ausgeben mind. eines im ersten Gehäuseteil 7 bevorrateten Medizinprodukts 6. Im Inneren des ersten Gehäuseteils 7 ist exemplarisch mind. ein einziges Medizinprodukt 6 eingelegt. Das zweite Gehäuseteil 8 definiert in seiner Hauptausdehnungsrichtung 12 eine gerade Zweitmittenachse 13 und bevorratet eine vorgegebene Masse oder ein vorgegebenes Volumen von reinigungsaktiver Flüssigkeit 2. Das erste Gehäuseteil 7 und das zweite Gehäuseteil 8 sind exemplarisch lösbar aneinander angeordnet, was bspw. durch eine Schraubverbindung 17 bewerkstelligt werden kann. Ferner ist der Bestückungsdurchgang 11, insb. in dessen Zusammenbauzustand, in Richtung der Erstmittenachse 10 und der Zweitmittenachse 13 axial zwischen dem ersten Gehäuseteil 7 und dem zweiten Gehäuseteil 8 angeordnet und vom zweiten Gehäuseteil 8 fluiddicht verschließbar gestaltet. Mit anderen Worten, das zweite Gehäuseteil 8 fungiert wie ein Deckel zum Verschließen des Bestückungsdurchgangs 11 des ersten Gehäuseteils 7.Der Multifunktionsspender 1 verfügt weiterhin über einen betätigbaren Flüssigkeitsspender 14 zum Ausgeben von reinigungsaktiven Flüssigkeiten 2, der am zweiten Gehäuseteil 8 fixiert ist, bspw. aufgeschraubt oder angeformt. In seiner Hauptausdehnungsrichtung 15 definiert der Flüssigkeitsspender 14 eine gerade Spendermittenachse 16. Wenn der Flüssigkeitsspender 14 insb. manuell betätigt ist, gibt er ein vorgegebenes Volumen oder eine vorgegebene Masse von reinigungsaktiver Flüssigkeit 2 aus dem zweiten Gehäuseteil 8 an einen Nutzer aus. Insb. die Mittenachse 5, die Spendermittenachse 16, die Erstmittenachse 10 und die Zweitmittenachse 13 sind zueinander parallel und zweckmäßigerweise koaxial ausgerichtet, um eine möglichst kompakte Gestalt des Multifunktionsspenders 1 zu erreichen. Das ist insb. dann von Vorteil, wenn das erste Gehäuseteil 7 und das zweite Gehäuseteil 8 rotationssymmetrisch bezüglich ihrer jeweiligen Achse sind.

Der besagte Flüssigkeitsspender 14 weist einen sich entlang der Spendermittenachse 16 erstreckenden rundzylindrischen Spenderschaft 18 zum Durchleiten, insb. Ansaugen, von reinigungsaktiven Flüssigkeiten 2 auf. Der Spenderschaft 18 hat einen Kreisrohrkörper 26 mit einem nicht mit seiner Mantelfläche gleichzusetzenden stofflichen Rohrmantel 27. Der Spenderschaft 18 ragt in Richtung der Spendermittenachse 16 lediglich abschnittsweise in das zweite Gehäuseteil 8 hinein. Einenends am Spenderschaft 18 ist ein von außen zugänglicher gehäuseaußenliegender knaufförmiger Spenderkopf 19 des Flüssigkeitsspenders 14 angeordnet, von dem ein bezüglich der Spendermittenachse 16 winkelig, insb. rechtwinkelig, wegragender innenhohler, mit dem Spenderschaft 18 fluidisch kommunizierender Spenderschnabel 20 zum Ausgeben von reinigungsaktiven Flüssigkeiten 2 angeordnet ist. Am Spenderkopf 20 kann ferner eine in Fig. 5 durch einen entsprechenden Pfeil angedeutete, insb. parallel bezüglich der Spendermittenachse 16 ausgerichtete, Betätigungskraft 21 eingeleitet werden, mittels der sich der Flüssigkeitsspender 14 betätigen lässt. Dadurch kann reinigungsaktive Flüssigkeit 2 aus dem zweiten Gehäuseteil 8 durch den Spenderschaft 18 hindurch zum Spenderschnabel 20 gefördert bzw. gepumpt und ausgegeben werden. Exemplarisch ist zwischen dem Spenderkopf 19 des Flüssigkeitsspenders 14 und dem Gehäuse 3 ein in Richtung der Spendermittenachse 16 mehrere Faltenberge werfender Faltenbalg 22 angeordnet.

Wie die Spenderköpfe 19 der Multifunktionsspender 1 der vorangehenden Ausführungsbeispiele ist auch der Spenderkopf 19 des vorliegenden Multifunktionsspender 1 drehbeweglich am Spenderschaft 18 angeordnet. Allerdings ist er lediglich zwischen zwei voneinander abweichenden Drehstellungen 29 um die Spendermittenachse 16 herum hin und her rotierbar. In Fig. 5 kann man erkennen, dass der Spenderkopf 19 drehbeweglich am Spenderschaft 18 oder am zweiten Gehäuseteil 8 angeordnet ist und dass der Flüssigkeitsspender 14, wenn der Spenderkopf 19 in die illustrierte als Sperrstellung 30 bezeichnete erste Drehstellung dieser beiden Drehstellungen 29 rotiert ist, in Richtung der Spendermittenachse 16 nicht betätigbar sondern bewegungsgesperrt ist. Dadurch kann mittels des Flüssigkeitsspenders 14 reinigungsaktive Flüssigkeit 2 nicht über den Spenderschnabel 20 zum Nutzer ausgegeben werden. Der Spenderkopf 19 definiert in dieser Drehstellung 29 eine Bezugswinkelposition 33, insb. exemplarisch 0°. In Fig. 5 ist ferner angedeutet, dass der Flüssigkeitsspender 14, wenn der Spenderkopf 19 in eine als Spendestellung 31 bezeichnete zweite Drehstellung dieser Drehstellungen 29 rotiert ist, in Richtung der Spendermittenachse 16 durch Beaufschlagung mit Betätigungskraft 21 des Spenderkopfs 19 in Richtung der Spendermittenachse 16 betätigbar ist. Dadurch kann mittels des Flüssigkeitsspenders 14 eine gewisse Masse bzw. ein gewisses Volumen an reinigungsaktiver Flüssigkeit 2 durch den Spenderschaft 18 gefördert, insb. gepumpt, und über den Spenderschnabel 20 zu einem Nutzer ausgegeben werden. In dieser Drehstellung 29 definiert der Spenderkopf 19 eine sogenannte Spendewinkelposition 34, welche gegenüber der besagten Bezugswinkelposition 33 im Winkelbereich von 0° bis +/-90° rechtwinkelig rotiert ist. Die am Spenderschaft 18 angeordneten Öffnungen 24 fungieren hier als Saugöffnungen.

Die Fig. 6 zeigt in einer perspektivischen Ansicht den Multifunktionsspender 1 aus Fig. 5, allerdings sind die beiden Gehäuseteile 7, 8 des Multifunktionsspenders 1 nun zueinander beabstandet angeordnet, so dass aus dem ersten Gehäuseteil 7 ein Medizinprodukt 6 entnommen werden oder eingelegt werden kann.

## Patentansprüche

1. Multifunktionsspender zum Ausgeben von reinigungsaktiven Flüssigkeiten,
- mit einem sich in einer Hauptausdehnungsrichtung (4) ersteckenden, mehrteiligen Gehäuse (3), das ein zum Aufnehmen von Medizinprodukten (6) geeignetes, einstückiges, erstes Gehäuseteil (7) und ein daran berührend angeordnetes, zum Aufnehmen von reinigungsaktiven Flüssigkeiten (2) geeignetes, einstückiges, zweites Gehäuseteil (8) aufweist,
- wobei das erste Gehäuseteil (7) eine Erstmittenachse (10) definiert, einen Bestückungsdurchgang (11) zum Durchführen von Medizinprodukten (6) aufweist und wobei durch den Bestückungsdurchgang (11) mindestens ein Medizinprodukt (6) in das erste Gehäuseteil (7) eingelegt ist,
- wobei das zweite Gehäuseteil (8) eine Zweitmittenachse (13) definiert und reinigungsaktive Flüssigkeit (2) bevorratet,
- wobei der Multifunktionsspender (1) weiterhin einen betätigbaren Flüssigkeitsspender (14) zum Ausgeben von reinigungsaktiven Flüssigkeiten (2) aufweist, der in seiner Hauptausdehnungsrichtung (15) eine Spendermittenachse (16) definiert und, wenn er betätigt ist, ein vorgegebenes Volumen oder eine vorgegebene Masse von reinigungsaktiver Flüssigkeit (2) aus dem zweiten Gehäuseteil (8) fördert und ausgibt,
- die Spendermittenachse (16), die Erstmittenachse (10) und die Zweitmittenachse (13) zueinander koaxial ausgerichtet sind,
wobei
- der Flüssigkeitsspender (14), wenn er manuell betätigt ist, ein vorgegebenes Volumen oder eine vorgegebene Masse von reinigungsaktiver Flüssigkeit (2) aus dem zweiten Gehäuseteil (8) fördert und entweder in das erste Gehäuseteil (7) zum Benetzen des darin angeordneten mind. einen Medizinprodukts (6) einleitet oder ausgibt,
- der Flüssigkeitsspender (14) einen sich entlang der Spendermittenachse (16) erstreckenden rundzylindrischen Spenderschaft (18) zum Durchleiten von reinigungsaktiven Flüssigkeiten (2) aufweist, wobei einenends am Spenderschaft (18) ein knaufförmiger Spenderkopf (19) des Flüssigkeitsspenders (14) angeordnet ist, von dem ein bezüglich der Spendermittenachse (16) winkelig wegragender innenhohler Spenderschnabel (20) zum Ausgeben von reinigungsaktiven Flüssigkeiten (2) angeordnet ist, wobei der Spenderschaft (18) das erste Gehäuseteil (7) und das zweite Gehäuseteil (8) jeweils zumindest abschnittsweise durchsetzt.

2. Multifunktionsspender nach Anspruch 1,
**dadurch gekennzeichnet, dass**
- das erste Gehäuseteil (7) und das zweite Gehäuseteil (8) lösbar aneinander angeordnet sind.

3. Multifunktionsspender nach Anspruch 1 oder 2,
**dadurch gekennzeichnet,**
- **dass** der Flüssigkeitsspender (14) berührend am ersten Gehäuseteil (7) fixiert ist, wobei der Spenderschaft (18) das erste Gehäuseteil (7) vollständig und das zweite Gehäuseteil (8) abschnittsweise durchsetzt, oder
- **dass** der Flüssigkeitsspender (14) berührend am zweiten Gehäuseteil (8) fixiert ist, wobei der Spenderschaft (18) das erste Gehäuseteil (7) abschnittsweise und das zweite Gehäuseteil (8) vollständig durchsetzt.

4. Multifunktionsspender nach einem der vorangehenden Ansprüche,
**dadurch gekennzeichnet, dass**
- der Flüssigkeitsspender (14) einen innerhalb des ersten Gehäuseteils (7) angeordneten Desinfektionsmittelausgabeabschnitt (23) zum Einleiten von reinigungsaktiven Flüssigkeiten (2) in das erste Gehäuseteil (7) zum Benetzen der im ersten Gehäuseteil (7) angeordneten Medizinprodukte (6) aufweist.

5. Multifunktionsspender nach Anspruch 4,
**dadurch gekennzeichnet,**
- **dass** der Desinfektionsmittelausgabeabschnitt (23) aus einer mehrere Sprühöffnungen (24) aufweisenden Öffnungsgruppe (25) gebildet ist.

6. Multifunktionsspender nach einem der vorangehenden Ansprüche,
**dadurch gekennzeichnet, dass**
- der Bestückungsdurchgang (11), insb. im Zusammenbauzustand des Multifunktionsspender 1, in Richtung der Erstmittenachse (10) und der Zweitmittenachse (13) axial zwischen dem ersten Gehäuseteil (7) und dem zweiten Gehäuseteil (8) angeordnet und vom zweiten Gehäuseteil (8) verschließbar oder verschlossen ist.

7. Multifunktionsspender nach einem der vorhergehenden Ansprüche,
**dadurch gekennzeichnet, dass**
- der Spenderkopf (19) drehbeweglich am Spenderschaft (18) angeordnet ist und zwischen mindestens drei voneinander abweichenden Drehstellungen (29) um die Spendermittenachse (16) herum hin und her rotiert werden kann,
- wobei der Flüssigkeitsspender (14), wenn der Spenderkopf (19) in eine als Sperrstellung (30) bezeichnete erste Drehstellung dieser Drehstellungen (29) rotiert ist, in welcher der Spenderkopf (19) eine Bezugswinkelposition (33) definiert, in Richtung der Spendermittenachse (16) nicht betätigbar ist, so dass mittels des Flüssigkeitsspenders (14) reinigungsaktive Flüssigkeit (2) weder über den Desinfektionsmittelausgabeabschnitt (23) in das erste Gehäuseteil (7) eingeleitet noch über den Spenderschnabel (20) ausgegeben ist,
- wobei der Flüssigkeitsspender (14), wenn der Spenderkopf (19) in eine als Spendestellung (31) bezeichnete zweite Drehstellung dieser Drehstellungen (29) rotiert ist, in welcher der Spenderkopf (19) eine im Winkelbereich von +10° bis +90° gegenüber der Bezugswinkelposition (33) abweichende Spendewinkelposition (34) definiert, in Richtung der Spendermittenachse (16) durch Beaufschlagung mit Betätigungskraft (21) des Spenderkopfs (19) betätigbar ist, so dass mittels des Flüssigkeitsspenders (14) reinigungsaktive Flüssigkeit (2) durch den Spenderschaft (18) gefördert und über den Spenderschnabel (20) ausgegeben ist,
- wobei der Flüssigkeitsspender (14), wenn der Spenderkopf (19) in eine als Benetzungsstellung (32) bezeichnete dritte Drehstellung dieser Drehstellungen (29) rotiert ist, in welcher der Spenderkopf (19) eine im Winkelbereich von -10° bis -90° gegenüber der Bezugswinkelposition (33) abweichende Benetzungswinkelposition (35) definiert, in Richtung der Spendermittenachse (16) durch Beaufschlagung mit Betätigungskraft (21) des Spenderkopfs (19) betätigbar ist, so dass mittels des Flüssigkeitsspenders (14) reinigungsaktive Flüssigkeit (2) aus dem zweiten Gehäuseteil (8) durch den Spenderschaft (18) des Flüssigkeitsspenders (14) hin zum Desinfektionsmittelausgabeabschnitt (23) durchgeleitet und das im ersten Gehäuseteil (7) angeordnete mind. eine Medizinprodukte (6) benetzt ist.

8. Multifunktionsspender nach einem der vorangehenden Ansprüche,
**dadurch gekennzeichnet, dass**
- das erste Gehäuseteil (7) und/oder das zweite Gehäuseteil (8) in eine Mittelkonsole (36) eines Kraftfahrzeuges integriert sind.

9. Multifunktionsspender nach einem der vorangehenden Ansprüche,
**dadurch gekennzeichnet,**
- **dass** im Inneren des ersten Gehäuseteils (7) ein Korb oder ein Gitter zum Aufnehmen und Spannen des mind. einen Medizinprodukts (6) angeordnet ist,
und/oder
- **dass** die besagten Sprühöffnungen (24) der Öffnungsgruppe (25) jeweils durch einen Zerstäuber gebildet sind, und/oder
- **dass** der besagte Bestückungsdurchgang (11) einen zum manuellen Einlegen und manuellen Entnehmen mind. eines Medizinprodukts (6) in das erste Gehäuseteil (7) geeigneten Runddurchgang (37) aufweist oder bildet, und/oder
- **dass** der besagte Bestückungsdurchgang (11) eine geschlitzte, insb. kreuz- oder sternförmig geschlitzte, Ausgabeeinrichtung oder einen einschlitzförmigen Durchgang (38) aufweist oder bildet, die jeweils zum Durchführen und Ausgeben mind. eines im ersten Gehäuseteil (7) bevorrateten Medizinprodukts (6) geeigneten sind.

## Claims

1. Multifunctional dispenser for dispensing detersive liquids,
- comprising a multipart housing (3) extending in a main direction of extension (4), which housing has a single-piece first housing part (7) suitable for receiving medical products (6) and a single-piece second housing part (8) suitable for receiving detersive liquids (2) and arranged in contact with the first housing part,
- wherein the first housing part (7) defines a first center axis (10) and has an insertion through-passage (11) for feeding through medical products (6) and wherein at least one medical product (6) is put into the first housing part (7) through the insertion through-passage (11),
- wherein the second housing part (8) defines a second center axis (13) and stores detersive liquid (2),
- wherein the multifunctional dispenser (1) further comprises an actuable liquid dispenser (14) for dispensing detersive liquids (2), which liquid dispenser defines, in the main direction of extension (15) thereof, a dispenser center axis (16) and, when the liquid dispenser is actuated, delivers and dispenses a specified volume or a specified mass of detersive liquid (2) from the second housing part (8),
- the dispenser center axis (16), the first center axis (10) and the second center axis (13) are coaxially orientated with respect to one another,
wherein
- the liquid dispenser (14), when it is manually actuated, delivers a specified volume or a specified mass of detersive liquid (2) from the second housing part (8) and either introduces it into the first housing part (7) in order to wet the at least one medical product (6) arranged therein, or dispenses it,
- the liquid dispenser (14) has a round-cylindrical dispenser shaft (18) extending along the dispenser center axis (16) for conveying through detersive liquids (2), wherein a knob-shaped dispenser head (19) of the liquid dispenser (14) is arranged at one end on the dispenser shaft (18), from which dispenser head an internally hollow dispenser spout (20) for dispensing detersive liquids (2) is arranged projecting away at an angle with respect to the dispenser central axis (16), wherein the dispenser shaft (18) passes through the first housing part (7) and the second housing part (8) in each case at least in portions.

2. Multifunctional dispenser according to claim 1,
**characterized in that**
- the first housing part (7) and the second housing part (8) are detachably arranged adjacent to one another.

3. Multifunctional dispenser according to claim 1 or 2,
**characterized in that**
- the liquid dispenser (14) is fixed in contact with the first housing part (7), wherein the dispenser shaft (18) passes through the first housing part (7) completely and passes through the second housing part (8) in portions, or
- the liquid dispenser (14) is fixed in contact with the second housing part (8), wherein the dispenser shaft (18) passes through the first housing part (7) in portions and passes through the second housing part (8) completely.

4. Multifunctional dispenser according to any of the preceding claims,
**characterized in that**
- the liquid dispenser (14) has a disinfectant dispensing portion (23) arranged within the first housing part (7) for introducing detersive liquids (2) into the first housing part (7) for wetting the medical products (6) arranged in the first housing part (7).

5. Multifunctional dispenser according to claim 4,
**characterized in that**
- the disinfectant dispensing portion (23) is formed from an opening group (25) having a plurality of spray openings (24).

6. Multifunctional dispenser according to any of the preceding claims,
**characterized in that**
- the insertion through-passage (11), in particular in the assembled state of the multifunctional dispenser 1, is arranged axially between the first housing part (7) and the second housing part (8) in the direction of the first center axis (10) and of the second center axis (13) and can be closed or is closed by the second housing part (8).

7. Multifunctional dispenser according to any of the preceding claims,
**characterized in that**
- the dispenser head (19) is arranged on the dispenser shaft (18) so as to be rotatably movable and can be rotated back and forth between at least three different rotational positions (29) about the dispenser center axis (16),
- wherein the liquid dispenser (14), when the dispenser head (19) is rotated into a first rotational position (29), designated as block position (30), of these rotational positions, in which position the dispenser head (19) defines a reference angle position (33), cannot be actuated in the direction of the dispenser center axis (16), so that detersive liquid (2) is neither introduced into the first housing part (7) via the disinfectant dispensing portion (23) nor dispensed via the dispenser spout (20), by means of the liquid dispenser (14),
- wherein the liquid dispenser (14), when the dispenser head (19) is rotated into a second rotational position (29), designated as dispensing position (31), of these rotational positions, in which position the dispenser head (19) defines a dispensing angle position (34) deviating with respect to the reference angle position (33) in an angular range of +10° to +90°, can be actuated in the direction of the dispenser center axis (16) by applying actuating force (21) to the dispenser head (19), so that detersive liquid (2) is delivered through the dispenser shaft (18) and dispensed via the dispenser spout (20), by means of the liquid dispenser (14),
- wherein the liquid dispenser (14), when the dispenser head (19) is rotated into a third rotational position (29), designated as wetting position (32), of these rotational positions, in which the dispenser head (19) defines a wetting angle position (35) deviating with respect to the reference angle position (33) in an angular range of -10° to -90°, can be actuated in the direction of the dispenser center axis (16) by applying actuating force (21) to the dispenser head (19), so that detersive liquid (2) from the second housing part (8) is conveyed through the dispenser shaft (18) of the liquid dispenser (14) to the disinfectant dispensing portion (23) and the at least one medical product (6) arranged in the first housing part (7) is wetted, by means of the liquid dispenser (14).

8. Multifunctional dispenser according to any of the preceding claims,
**characterized in that**
- the first housing part (7) and/or the second housing part (8) are integrated into a center console (36) of a motor vehicle.

9. Multifunctional dispenser according to any of the preceding claims,
**characterized in that**
- a basket or a grating for receiving and gripping the at least one medical product (6) is arranged inside the first housing part (7), and/or
- said spray openings (24) of the opening group (25) are each formed by an atomizer, and/or
- said insertion through-passage (11) has or forms a circular through-passage (37) suitable for manually putting in and manually removing at least one medical product (6) into the first housing part (7), and/or
- said insertion through-passage (11) has or forms a slotted, in particular slotted in a cross or star shape, dispensing device or a single-slotted through-passage (38), each of which is suitable for feeding through and dispensing at least one medical product (6) stored in the first housing part (7).

## Revendications

1. Distributeur multifonction destiné à la distribution de liquides à action de nettoyage,
- comportant un boîtier (3) en plusieurs parties s'étendant dans une direction d'extension principale (4), qui présente une première partie de boîtier (7) en une seule pièce adaptée pour recevoir des dispositifs médicaux (6) et une seconde partie de boîtier (8) d'une seule pièce, agencée en contact avec celle-ci et adaptée pour recevoir des liquides à action de nettoyage (2),
- dans lequel la première partie de boîtier (7) définit un axe central initial (10), présente un passage de chargement (11) destiné au passage de dispositifs médicaux (6) et dans lequel au moins un dispositif médical (6) est inséré dans la première partie de boîtier (7) à travers le passage de chargement (11),
- dans lequel la seconde partie de boîtier (8) définit un axe central second (13) et stocke un liquide à action de nettoyage (2),
- dans lequel le distributeur multifonction (1) présente en outre un distributeur de liquide (14) actionnable destiné à la distribution de liquides à action de nettoyage (2), qui définit, dans sa direction d'extension principale (15), un axe central de distributeur (16) et qui, lorsqu'il est actionné, achemine et distribue un volume prédéfini ou une masse prédéfinie de liquide à action de nettoyage (2) à partir de la seconde partie de boîtier (8),
- dans lequel l'axe central de distributeur (16), l'axe central initial (10) et l'axe central second (13) sont alignés de manière coaxiale les uns par rapport aux autres,
dans lequel
- le distributeur de liquide (14), lorsqu'il est actionné manuellement, achemine un volume prédéfini ou une masse prédéfinie de liquide à action de nettoyage (2) depuis la seconde partie de boîtier (8) et soit l'introduit dans la première partie de boîtier (7) pour mouiller l'au moins un dispositif médical (6) qui est agencé dedans, soit le distribue,
- le distributeur de liquide (14) présente un arbre de distributeur (18) de forme cylindrique circulaire s'étendant le long de l'axe central de distributeur (16), destiné à l'acheminement de liquides à action de nettoyage (2), dans lequel une tête de distributeur (19) en forme de pommeau du distributeur de liquide (14) est agencée à une extrémité de l'arbre de distributeur (18), à partir de laquelle est agencé, faisant saillie en s'éloignant en formant un angle par rapport à l'axe central de distributeur (16), un bec de distributeur (20) creux à l'intérieur, destiné à la distribution de liquides à action de nettoyage (2), dans lequel l'arbre de distributeur (18) traverse respectivement au moins par sections la première partie de boîtier (7) et la seconde partie de boîtier (8).

2. Distributeur multifonction selon la revendication 1,
**caractérisé en ce que**
- la première partie de boîtier (7) et la seconde partie de boîtier (8) sont agencées l'une sur l'autre de manière amovible.

3. Distributeur multifonction selon la revendication 1 ou 2,
**caractérisé en ce**
- **que** le distributeur de liquide (14) est fixé par contact à la première partie de boîtier (7), dans lequel l'arbre de distributeur (18) traverse entièrement la première partie de boîtier (7) et la seconde partie de boîtier (8) par sections, ou
- **que** le distributeur de liquide (14) est fixé par contact à la seconde partie de boîtier (8), dans lequel l'arbre de distributeur (18) traverse la première partie de boîtier (7) par sections et entièrement la seconde partie de boîtier (8).

4. Distributeur multifonction selon l'une des revendications précédentes,
**caractérisé en ce que**
- le distributeur de liquide (14) présente une section de distribution d'agent désinfectant (23), agencée à l'intérieur de la première partie de boîtier (7), destinée à l'introduction de liquides à action de nettoyage (2) dans la première partie de boîtier (7) afin de mouiller les dispositifs médicaux (6) agencés dans la première partie de boîtier (7).

5. Distributeur multifonction selon la revendication 4,
**caractérisé en ce**
- **que** la section de distribution d'agent désinfectant (23) est formée d'un groupe d'orifices (25) présentant plusieurs orifices de pulvérisation (24).

6. Distributeur multifonction selon l'une des revendications précédentes,
**caractérisé en ce que**
- le passage de chargement (11), notamment lorsque le distributeur multifonction 1 est assemblé, est agencé, dans la direction de l'axe central initial (10) et de l'axe central second (13) axialement entre la première partie de boîtier (7) et la seconde partie de boîtier (8) et peut être fermé ou est fermé par la seconde partie de boîtier (8).

7. Distributeur multifonction selon l'une des revendications précédentes,
**caractérisé en ce que**
- la tête de distributeur (19) est agencée de manière à pouvoir se déplacer en rotation sur l'arbre de distributeur (18) et peut effectuer un mouvement de rotation dans les deux sens autour de l'axe central de distributeur (16) entre au moins trois positions de rotation (29) différentes les unes des autres,
- dans lequel le distributeur de liquide (14), lorsque la tête de distributeur (19) est entraînée en rotation dans une première position de rotation, désignée comme position de blocage (30), parmi ces positions de rotation (29), dans laquelle la tête de distributeur (19) définit une position angulaire de référence (33), ne peut pas être actionnée dans la direction de l'axe central de distributeur (16), de sorte que du liquide à action de nettoyage (2) ne peut être ni introduit dans la première partie de boîtier (7) par l'intermédiaire de la section de distribution d'agent désinfectant (23) ni distribué par le bec de distributeur (20) par le biais du distributeur de liquide (14),
- dans lequel le distributeur de liquide (14), lorsque la tête de distributeur (19) est entraînée en rotation dans une deuxième position de rotation, désignée comme position de distribution (31), parmi ces positions de rotation (29), dans laquelle la tête de distributeur (19) définit une position angulaire de distributeur (34) s'écartant de la position angulaire de référence (33) d'une plage angulaire de +10° à +90°, peut être actionnée en direction de l'axe central de distributeur (16) par l'application d'une force d'actionnement (21) sur la tête de distributeur (19), de sorte que, par le biais du distributeur de liquide (14), un liquide à action de nettoyage (2) est acheminé à travers l'arbre de distributeur (18) et distribué par le bec de distributeur (20),
- dans lequel le distributeur de liquide (14), lorsque la tête de distributeur (19) est entraînée en rotation dans une troisième position de rotation, désignée comme position de mouillage (32), parmi ces positions de rotation (29), dans laquelle la tête de distributeur (19) définit une position angulaire de mouillage (35) s'écartant de la position angulaire de référence (33) d'une plage angulaire de -10° à -90°, peut être actionnée en direction de l'axe central de distributeur (16) par l'application d'une force d'actionnement (21) sur la tête de distributeur (19), de sorte que, par le biais du distributeur de liquide (14), le liquide à action de nettoyage (2) est acheminé depuis la seconde partie de boîtier (8) vers la section de distribution d'agent désinfectant (23) à travers l'arbre de distributeur (18) du distributeur de liquide (14), et qu'au moins un dispositif médical (6) agencé dans la première partie de boîtier (7) est mouillé.

8. Distributeur multifonction selon l'une des revendications précédentes,
**caractérisé en ce que**
- la première partie de boîtier (7) et/ou la seconde partie de boîtier (8) sont intégrées dans une console centrale (36) d'un véhicule automobile.

9. Distributeur multifonction selon l'une des revendications précédentes,
**caractérisé en ce**
- **que,** à l'intérieur de la première partie du boîtier (7) est agencé(e) un panier ou une grille destiné(e) à la réception et au maintien en place d'au moins un dispositif médical (6), et/ou
- **que** lesdits orifices de pulvérisation (24) du groupe d'orifices (25) sont respectivement formés par un atomiseur, et/ou
- **que** ledit passage de chargement (11) présente ou forme un passage circulaire (37) adapté à l'insertion manuelle et au retrait manuel d'au moins un dispositif médical (6) dans la première partie de boîtier (7), et/ou
- **que** ledit passage de chargement (11) présente ou forme un dispositif de distribution fendu, notamment en forme de croix ou d'étoile, ou un passage (38) en forme de fente unique, qui sont respectivement adaptés pour le passage et la distribution d'au moins un dispositif médical (6) stocké dans la première partie de boîtier (7).
